# EUROPEAN PATENT APPLICATION

(11) **EP 4 726 018 A1**
(43) Date of publication of application: **15.04.2026**
(21) Application number: 25207983.5
(22) Date of filing: 10.10.2025
(51) Int. Cl.: C11B 9/00, A61K 8/03, A61K 8/19

(54) **A LIQUID PERFUME HAVING THREE LAYERS AND THREE COLORS AND A MANUFACTURING METHOD THEREOF**

(30) Priority: 14.10.2024 CN 202411430515
(71) Applicant: Anhui Fenyuan Aromatic Technology Co., Ltd., Wuhu, Anhui 241000 (CN)
(72) Inventor: TIAN, Dong, Wuhu City, Anhui Province, 241000 (CN); TU, Hongjian, Wuhu City, Anhui Province, 241000 (CN); WU, Qianyu, Wuhu City, Anhui Province, 241000 (CN); LI, Mingxia, Wuhu City, Anhui Province, 241000 (CN)
(74) Representative: Meyer-Dulheuer MD Legal Patentanwälte PartG mbB

(57) **Abstract**

A liquid perfume having three layers and three colors comprises a perfume liquor having an upper layer, a middle layer and a lower layer; the upper layer is an oil phase layer comprising an oil phase solvent and an upper-layer pigment which are dissolved into each other; the middle layer is a water phase layer comprising middle-layer deionized water, a middle-layer water phase solvent and a middle-layer pigment which are dissolved into each other; the lower layer is a water phase layer comprising lower-layer deionized water, a lower-layer water phase solvent, a lower-layer pigment and salts which are dissolved into each other ; the upper-layer, the middle-layer and the lower-layer each have one kind of different color due to their respective layer pigments ; at least one of the upper layer, the middle layer and the lower layer has an essence. Thus, the whole liquid perfume has rich visual effects with three colors and three layers and emits fragrance, and can satisfy the consumers' diversified needs. The present invention also discloses a manufacturing method thereof.

## Description

### BACKGROUND

### Technical Field

The present invention relates to a technical field of perfumes, in particular to a liquid perfume having three layers and three colors and a manufacturing method of the liquid perfume.

### Description of Related Art

With the improvement of people's living standards, consumers' pursuit for the quality of life and spiritual pleasure is increasing. Liquid perfumes are often unique in fragrance, and some of them have functional components, which can improve the environment odor, elevate mood, relieve stress and the like and satisfy consumers' needs for the improvement in the quality of life.

At present, most volatile perfumes are single-phase transparent liquid or double-layer liquid. For example, a Chinese invention patent CN108102784B disclosed a double-color perfume oil and a manufacturing method thereof, the double-color perfume oil includes a component A and a component B; the component A includes the following raw materials in parts by weight: 75 to 90 parts of an oily solvent, 10 to 25 parts of an oily essence and 0.02 to 0.5 parts of an oily pigment; the component B includes the following raw materials in parts by weight: 75 to 90 parts of water, 10 to 25 parts of an aqueous essence and 0.02 to 0.5 parts of an aqueous pigment; the oily essence and the aqueous essence are essences with the same fragrance and different water-oil properties; the oily solvent includes dipropyleneglycol methyl ether acetate and isoparaffin; and, the oily pigment and the aqueous pigment have different colors. In the double-color perfume oil and the preparation method thereof provided by this invention, the component A and the component B are not dissolvable, and the oily pigment and the aqueous pigment have different colors, so that the perfume oil has two colors layered up and down, thus enriching the color of perfume oils in the market and bringing new visual effects and experience to users.

However, the above patent has not disclosed how to prepare a liquid perfume having three layers and three colors.

### SUMMARY

It is a first object of the present invention to provide a liquid perfume having three layers and three colors.

It is a second object of the present invention to provide a manufacturing method for the three-color three-layer liquid perfume.

For achieving the first object, a liquid perfume having three layers and three colors, comprises a perfume liquor having an upper layer, a middle layer and a lower layer; wherein, the upper layer is an oil phase layer comprising an oil phase solvent and an upper-layer pigment which are dissolved into each other; the middle layer is a water phase layer comprising middle-layer deionized water, a middle-layer water phase solvent and a middle-layer pigment which are dissolved into each other; the lower layer is a water phase layer comprising lower-layer deionized water, a lower-layer water phase solvent, a lower-layer pigment and salts which are dissolved into each other; the upper-layer, the middle-layer and the lower-layer each have one kind of different color due to their respective layer pigments; a density of the lower-layer water phase solvent is greater than a density of the middle-layer water phase solvent, which is greater than a density of the upper-layer oil phase solvent; a polarity of the lower layer is greater than a polarity of the middle layer, which is greater than a polarity of the upper layer; and, at least one of the upper layer, the middle layer and the lower layer has an essence.

Preferably, in the upper layer, the oil phase solvent comprises at least one of isoparaffin, dipropyleneglycol methyl ether acetate, caprylic/capric triglyceride, isopropyl myristate and white oil; and/or, the middle-layer water phase solvent comprises 3-methoxy-3-methyl-1-butanol, and further comprises at least one of dipropyleneglycol methyl ether ethyl ester, dipropylene glycol methyl ether, dipropyleneglycol dimethyl ether and ethanol; and/or, the lower-layer water phase solvent comprises at least one of 3-methoxy-3-methyl-1-butanol, dipropyleneglycol methyl ether ethyl ester, dipropylene glycol methyl ether, dipropyleneglycol dimethyl ether and ethanol; and/or, the salts comprises at least one of potassium chloride, anhydrous sodium sulfate and sodium chloride; and/or, the essence comprises a natural essence and/or a synthetic essence.

To prevent the pigment from fading and discoloring, preferably, the upper layer further comprises an oil-soluble pigment stabilizer, and the oil-soluble pigment stabilizer is dissolved in the oil phase solvent to prevent the upper-layer pigment from fading and discoloring;
and/or, the middle layer further comprises a water-soluble pigment stabilizer, and the water-soluble pigment stabilizer is dissolved in the middle-layer water phase solvent to prevent the middle-layer pigment from fading and discoloring;
and/or, the lower layer further comprises a water-soluble pigment stabilizer, and the water-soluble pigment stabilizer is dissolved in the lower-layer water phase solvent to prevent the lower-layer pigment from fading and discoloring.

Preferably, the oil-soluble pigment stabilizer comprises at least one of octyl methoxycinnamate, butyl methoxydibenzoylmethane and octyl salicylate;
and/or, the water-soluble pigment stabilizer comprises at least one of octyl methoxycinnamate, butyl methoxydibenzoylmethane, PPG-26-buteth-26, octyl salicylate and PEG-40-hydrogenated castor oil.

To contain the perfume liquor, preferably, the liquid perfume comprises a containing bottle for containing the perfume liquor. The containing bottle may be have an opening for facilitating the volatilization of the perfume liquor. The containing bottle may also be sealed to contain the perfume liquor. In this case, the perfume liquor in the containing bottle is stored as a supplementary liquor;

Preferably, the containing bottle has an opening at a top of the containing bottle, and the liquid perfume further comprises a volatile stick which is inserted into the opening and immersed in the lower layer of the perfume liquor;
the containing bottle is made of a transparent material.

Thus, the containing bottle has an opening, for facilitating the placement of the volatile stick and the emission of the fragrance. The containing bottle is made of a transparent material (e.g., transparent glass or transparent plastics), for facilitating users to see the color of the perfume liquor. The volatile stick plays two roles, i.e., absorbing the perfume liquor upward, and adsorbing the salt on the volatile stick (e.g., rattan) after use for a period of time. Near the volatile stick outside the containing bottle, the air flow is large, and more water is evaporated. This part of the volatile stick is relatively dry, so that a dense white crystalline substance is adhered to the volatile stick outside the containing bottle, so that another color is added. Thus, the whole liquid perfume product is richer in color, increasing different visual effects and improving user experience.

For achieving the second object, a manufacturing method for the liquid perfume having three layers and three colors, comprises the following steps:
step 1:
   dissolving a salt in total deionized water, adding a water-soluble pigment, and mixing uniformly to obtain a first solution; and
   using a total water phase solvent as a second solution;
step 2:
   mixing an oil phase solvent with an oil-soluble pigment uniformly to obtain a third solution, the water-soluble pigment and the oil-soluble pigment having different colors;
   if the essence is water-soluble, the essence is added in the step 1; and, if the essence is oil-soluble, the essence is added in the step 2;
step 3:
   according to the volume required by each layer of the final product liquid perfume, determining the volume required by each of the first solution, the second solution and the third solution, mixing and shaking the first solution and the second solution uniformly, standing to clarification, adding the third solution and further mixing and shaking uniformly, and standing to clarification to obtain the upper layer, the middle layer and the lower layer;
   a total deionized water comprises the middle-layer deionized water and the lower-layer deionized water;
   a total water phase solvent comprises the middle-layer water phase solvent and the lower-layer water phase solvent;
   the water-soluble pigment finally enters the middle layer and the lower layer, that is, the water-soluble pigment comprises part of the middle-layer pigment and part of the lower-layer pigment; and
   the oil-soluble pigment finally enters the upper layer and the middle layer, that is, the oil-soluble pigment comprises part of the upper-layer pigment and part of the middle-layer pigment.

To present the pigment from fading and discoloring, preferably, during preparation of the first solution in the step 1, after the water-soluble pigment is added, a water-soluble pigment stabilizer for preventing the water-soluble pigment from fading and discoloring is also added; and
in the step 3, after the oil phase solvent and the oil-soluble pigment are added, an oil-soluble pigment stabilizer for preventing the oil-soluble pigment from fading and discoloring is also added.

To ensure the layering of the first solution and the second solution after being mixed, components by volume percentage are:
25ml to 40ml of total deionized water;
5g to 8g of sodium chloride;
the total water phase solvent comprises:
10ml to 35ml of 3-methoxy-3-methyl-1-butanol;
3ml to 15ml of dipropylene glycol methyl ether.

Preferably, the essence is oil-soluble and added during the preparation of the third solution in the step 2, and the third solution further comprises the following components by volume percentage:
2ml to 15ml of ethanol;
10ml to 25ml of the essence.

Ethanol can remove foams, which is advantageous for clarification. Otherwise, there are some foams in the process of mixing the first solution, the second solution and the third solution.

To achieve the almost equal and balanced volatilization rate of the three layers, preferably, the oil phase solvent is 10ml or 25ml of isoparaffin, or dipropyleneglycol methyl ether acetate, or caprylic/capric triglyceride, or isopropyl myristate, or white oil. Thus, the liquid perfume having three layers and three colors is still maintained after the liquid perfume is volatilized for a period of time. If the volatilization rate of a certain layer is too high, after use for a period of time, the height ratio of the three layers will be uncoordinated and will be changed, so that the three-layer state may not be maintained stably.

Compared with the prior art, the liquid perfume of the present invention has the following advantages. The lower-layer water phase solvent, the middle-layer water phase solvent and the upper-layer oil phase solvent are different in density, and the upper, middle and lower layers are different in polarity, so it is ensured that the perfume liquor is divided into three layers. The corresponding pigment is dissolved in the corresponding layer, so that the upper, middle and lower layers present different colors. The essence makes the liquid perfume fragrant, and the fragrance can be expanded, thus elevating the user's mood and improving the environment. Thus, the whole liquid perfume has rich visual effects with three colors and three layers and emits fragrance, and can satisfy the consumers' diversified needs.

### BRIEF DESCRIPTION OF THE DRAWINGS

The patent of application file contains at least one drawing executed in color. Copies of this patent or patent application publication with color drawing(s) will be provided by the Office upon request and payment of the necessary fee.
Fig. 1 is a picture of a liquid perfume having three layers and three colors according to Embodiment 1 of the present invention;
Fig. 2 is a picture of a liquid perfume having three layers and three colors according to Embodiment 2 of the present invention.

### DESCRIPTION OF THE EMBODIMENTS

The present invention will be further described below in detail by embodiments with reference to the accompanying drawings.

In this embodiment, the liquid perfume having three layers and three colors, comprises a perfume liquor which has an upper layer, a middle layer and a lower layer.

The upper layer is an oil phase layer comprising an oil phase solvent and an upper-layer pigment which are dissolved into each other; the upper layer further comprises an oil-soluble pigment stabilizer, and the oil-soluble pigment stabilizer is dissolved in the oil phase solvent to prevent the upper-layer pigment from fading and discoloring.

The middle layer is a water phase layer comprising middle-layer deionized water, a middle-layer water phase solvent and a middle-layer pigment which are dissolved into each other; the middle layer further comprises a water-soluble pigment stabilizer, and the water-soluble pigment stabilizer is dissolved in the middle-layer water phase solvent to prevent the middle-layer pigment from fading and discoloring.

The lower layer is a water phase layer comprising lower-layer deionized water, a lower-layer water phase solvent, a lower-layer pigment and salts which are dissolved into each other; the lower layer further comprises a water-soluble pigment stabilizer, and the water-soluble pigment stabilizer is dissolved in the lower-layer water phase solvent to prevent the lower-layer pigment from fading and discoloring.

The upper-layer, the middle-layer and the lower-layer each have one kind of different color due to their respective layer pigments.

A density of the lower-layer water phase solvent is greater than a density of the middle-layer water phase solvent, which is greater than a density of the upper-layer oil phase solvent.

A polarity of the lower layer is greater than a polarity of the middle layer, which is greater than a polarity of the upper layer. When the substances in the following manufacturing method are specifically determined, the polarity can be determined by theoretical analysis, which belongs to the prior art and will not be specifically described here.

At least one of the upper layer, the middle layer and the lower layer has an essence. Thus, the perfume liquor can emit fragrance. An oil-soluble essence or a water-soluble essence can be selected as required. If the oil-soluble essence is used, the oil-soluble essence is located in the upper-layer oil phase layer; and, if the water-soluble essence is used, the water-soluble essence is partially located in the middle layer and partially located in the lower layer.

The specific components of the oil phase solvent, the middle-layer water phase solvent and the lower-layer water phase solvent are given below.

In the upper layer, the oil phase solvent comprises isoparaffin.

The middle-layer water phase solvent comprises 3-methoxy-3-methyl-1-butanol, and further comprises at least one of dipropyleneglycol methyl ether ethyl ester, dipropylene glycol methyl ether, dipropyleneglycol dimethyl ether and ethanol, which are specifically coordinated as required.

The lower-layer water phase solvent comprises at least one of 3-methoxy-3-methyl-1-butanol, dipropyleneglycol methyl ether ethyl ester, dipropylene glycol methyl ether, dipropyleneglycol dimethyl ether and ethanol, which are specifically coordinated as required.

The salts comprise at least one of potassium chloride, anhydrous sodium sulfate and sodium chloride, which are specifically selected as required. The addition of the salt is advantageous to affect the solubility and compatibility of the water phase solvent in the deionized water, so that the water phase is layered in the lower layer and the middle layer.

The essence comprises a natural essence and/or a synthetic essence. The natural essence can be rose oil, jasmine oil, rosa multiflora oil, or magnolia oil, etc. The synthetic essence is, e.g., propyl acetate, octyl acetate, citric acid, water-soluble peppermint essence, water-soluble food flavoring pineapple essence, etc.

The oil-soluble pigment is, e.g., red, yellow, green, blue, orange, golden, purple, etc.; and, the water-soluble pigment is, e.g., red, yellow, orange, green, blue, purples, golden, etc. According to the three layers and three colors finally required by the perfume liquor, the corresponding pigment toners are coordinated. The pigment toners belong to the prior art, and can be, for example, purchased from Sensient Technology Corporation (China).

The oil-soluble pigment stabilizer comprises at least one of octyl methoxycinnamate, butyl methoxydibenzoylmethane and octyl salicylate. The specific oil-soluble pigment stabilizer may be a mixture of ctyl methoxycinnamate, butyl methoxydibenzoylmethane and octyl salicylate, which are purchased from Sensient Technology Corporation (China) (Model No.: COVABSORB EW).

The water-soluble pigment stabilizer comprises at least one of octyl methoxycinnamate, butyl methoxydibenzoylmethane, PPG-26-buteth-26, octyl salicylate and PEG-40-hydrogenated castor oil. The specific water-soluble pigment stabilizer may be a mixture of octyl methoxycinnamate, butyl methoxydibenzoylmethane, PPG-26-buteth-26, octyl salicylate and PEG-40-hydrogenated castor oil, which are purchased from Sensient Technology Corporation (China) (Model No.: COVABSORB).

The liquid perfume comprises a containing bottle for containing the perfume liquor, and a volatile stick. The containing bottle has an opening at a top of the containing bottle, and the volatile stick is inserted into the opening and immersed in the lower layer of the perfume liquor, that is, the volatile stick is higher than the liquid level of the perfume liquor. The volatile stick is a rattan or artificial fiber stick. The containing bottom is made of a transparent material, e.g., transparent glass or transparent plastics, so that it is convenient to see the three colors and three layers of the perfume liquor from the outside of the containing bottom.

A manufacturing method for the liquid perfume having three layers and three colors, comprises the following steps:
Step 1:
   dissolving the salts in total deionized water, adding the water-soluble pigment, adding the water-soluble pigment stabilizer for preventing the water-soluble pigment from fading, and discoloring and mixing uniformly to obtain a first solution;
   using a total water phase solvent as a second solution.
Step 2:
   mixing the oil phase solvent, the oil-soluble pigment and the oil-soluble pigment stabilizer for preventing the oil-soluble pigment from fading, and discoloring uniformly to obtain a third solution, the water-soluble pigment and the oil-soluble pigment have different colors;
   if the essence is water-soluble, the essence is added in the step 1 (the essence can be added during the preparation of the first solution or during the preparation of the second solution); and, if the essence is oil-soluble, the essence is added in the step 2.
Step 3:
   according to the volume required by each layer of the final product liquid perfume, determining the volume required by each of the first solution, the second solution and the third solution, mixing and shaking the first solution and the second solution uniformly, standing to clarification, adding the third solution and further mixing and shaking uniformly, and standing to clarification to obtain the upper layer, the middle layer and the lower layer;
   a total deionized water comprises the middle-layer deionized water and the lower-layer deionized water. That is, the total deionized water in the step 1 enters partially the middle layer and partially the lower layer in the final liquid perfume;
   a total water phase solvent comprises the middle-layer water phase solvent and the lower-layer water phase solvent. The total water phase solvent in the step 1 enters partially the middle layer and partially the lower layer in the final liquid perfume.

The water-soluble pigment finally enters the middle layer and the lower layer, that is, the water-soluble pigment comprises part of the middle-layer pigment and part of the lower-layer pigment.

The oil-soluble pigment finally enters the upper layer and the middle layer, that is, the oil-soluble pigment comprises part of the upper-layer pigment and part of the middle-layer pigment.

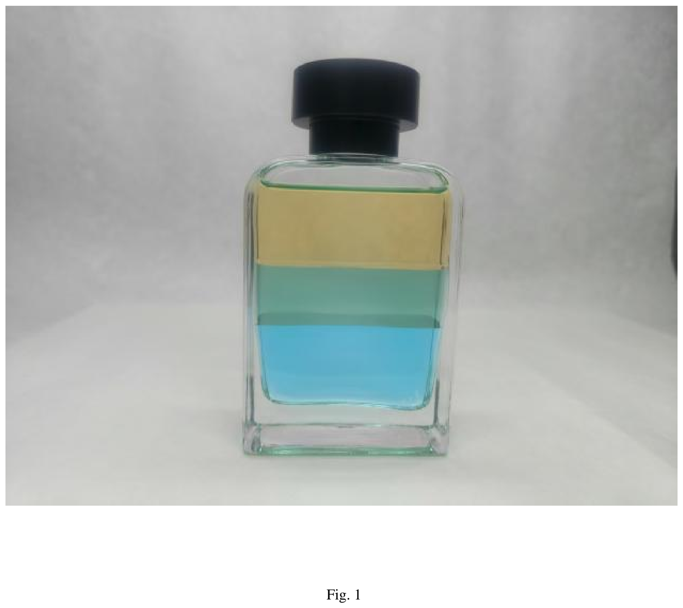

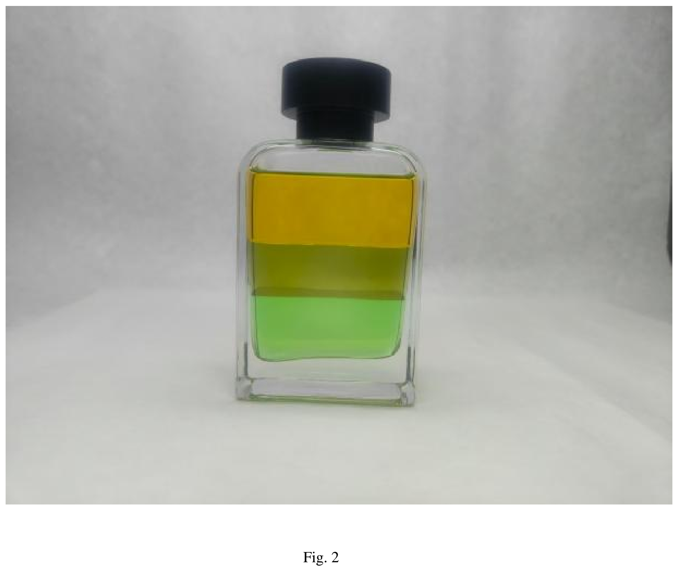

In other words, due to the different solubility and coloring of pigments in different solvents, the water-soluble pigment added in the step 1 will finally partially reach the middle layer and partially reach the lower layer, and the oil-soluble pigment added in the step 2 will finally be mostly located in the upper layer and be less located in the middle layer, so that the upper, middle and lower layers of the final liquid perfume present different colors. The pigments will be described below by taking FIGS. 1 and 2 as examples.

For example, in FIG. 1, the water-soluble pigment added in the step 1 is blue and enters the middle layer and the lower layer in the final product, and the oil-soluble pigment added in the step 2 is yellow and enters the upper layer (more in the upper layer) and the middle layer in the final product, so that the upper layer of the final perfume liquor is yellow, the middle layer is light blue and greenish (because the blue and yellow pigments are combined) and the lower layer is dark blue.

For another example, in FIG. 2, the water-soluble pigment added in the step 1 is green and enters the middle layer and the lower layer in the final product, and the oil-soluble pigment added in the step 2 is yellow and enters the upper layer (more in the upper layer) and the middle layer in the final product, so that the upper layer of the final perfume liquor is yellow, the middle layer is light green and yellowish (because the yellow and green pigments are combined) and the lower layer is dark green.

The components by proportion are:
25ml to 40ml of total deionized water;
5g to 8g of sodium chloride;
the total water phase solvent comprises:
10ml to 35ml of 3-methoxy-3-methyl-1-butanol;
3ml to 15ml of dipropylene glycol methyl.

The essence is oil-soluble and added during the preparation of the third solution in the step 2, and the third solution further comprises the following components by volume percentage:
2ml to 15ml of ethanol;
10ml to 25ml of the essence.

The oil phase solvent is 10ml - 25ml of isoparaffin, or dipropyleneglycol methyl ether acetate, or caprylic/capric triglyceride, or isopropyl myristate, or white oil.

The specific parameters of the embodiments are shown in Table 1.

| | Compon ent | Embodi ment 1 | Embodi ment 2 | Embodime nt 3 | Embodim ent 4 | Embodi ment 5 | Embodi ment 6 |
|---|---|---|---|---|---|---|---|
| First Soluti on | Several grams of the salt | Sodium chloride 5 g | Sodium chloride 6 g | Sodium chloride 8 g | Sodium chloride 8 g | Sodium chloride 8 g | Sodium chloride 8 g |
| | Total deionize d water | Deionize d water 25ml | Deionize d water 30 ml | Deionized water 40 ml | Deionized water 40 ml | Deioniz ed water 40 ml | Deionize d water 40 ml |
| | Total water-sol uble pigment | Using toner, blue toner | Using toner, green toner | Using toner, blue toner | Using toner, blue toner | Using toner, green toner | Using toner, red toner |
| | Water-so luble pigment stabilizer | Purchase d from Sensient Technology Corporat ion (China) (Model No. COVAB SORB) | Purchase d from Sensient Technology Corporat ion (China) (Model No. COVAB SORB) | Purchased from Sensient Technology Corporatio n (China) (Model No. COVABS ORB) | Purchased from Sensient Technology Corporati on (China) (Model No. COVABS ORB) | Purchas ed from Sensient Technology Corpora tion (China) (Model No. COVAB SORB) | Purchase d from Sensient Technology Corporat ion (China) (Model No. COVAB SORB) |
| | Compon ent | Embodi ment 1 | Embodi ment 2 | Embodime nt 3 | Embodim ent 4 | Embodi ment 5 | Embodi ment 6 |
| Secon d Soluti on | Total water-ph ase solvent | 3-metho xy-3-met hyl-1-but anol 20 ml | 3-metho xy-3-met hyl-1-but anol 30 ml | 3-methoxy -3-methyl-1-butanol 35 ml | 3-methox y-3-meth yl-1-buta nol 20 ml | / | / |
| | | Dipropyl ene glycol methyl ether 3 ml | Dipropyl ene glycol methyl ether 10 ml | Dipropyle ne glycol methyl ether 15 ml | Dipropyle ne glycol methyl ether 3 ml | Dipropy lene glycol methyl ether 10 ml | Dipropyl ene glycol methyl ether 15 ml |
| | Essence | / | / | / | / | Water-s oluble essence, specific ally water-so luble food flavorin g pineapple essence 10ml | Water-so luble essence, specifica lly water-sol uble peppermi nt essence 25ml |
| Comp onent | Compon ent | Embodi ment 1 | Embodi ment 2 | Embodime nt 3 | Embodim ent 4 | Embodi ment 5 | Embodi ment 6 |
| Third Soluti on | Oil phase solvent | Isoparaff in 10 ml | Isoparaff in 20 ml | Isoparaffin 25 ml | Dipropyle neglycol methyl ether acetate 10 ml | Dipropy leneglyc ol methyl ether acetate 20 ml | Dipropyl eneglyco l methyl ether acetate 25 ml |
| | Oil-solub le pigment | Using toner, yellow toner | Using toner, yellow toner | Using toner, yellow toner | Using toner, purple toner | Using toner, purple toner | Using toner, golden toner |
| | Essence | Oil-solub le essence, specifica lly rose oil 10ml | Oil-solub le essence, specifica lly rose oil 20 ml | Oil-solubl e essence, specificall y jasmine oil 25 ml | Oil-solubl e essence, specificall y jasmine oil 10 ml | / | / |
| | Ethanol | Ethanol 2 ml | Ethanol 10 ml | Ethanol 15 ml | Ethanol 2 ml | Ethanol 8 ml | Ethanol 15 ml |
| | Oil-solub le pigment stabilizer | Purchase d from Sensient Technolo gy Corporat ion (China) (Model No. COVAB SORB EW) | Purchase d from Sensient Technolo gy Corporat ion (China) (Model No. COVAB SORB EW) | Purchased from Sensient Technolog y Corporatio n (China) (Model No. COVABS ORB EW) | Purchased from Sensient Technolo gy Corporati on (China) (Model No. COVABS ORB EW) | Purchas ed from Sensient Technol ogy Corpora tion (China) (Model No. COVAB SORB EW) | Purchase d from Sensient Technolo gy Corporat ion (China) (Model No. COVAB SORB EW) |
| / | Are there three color and three layers? | Yes | Yes | Yes | Yes | Yes | Yes |
| | Layering stability duration | Within 12 h | Within 12 h | Within 12 h | Within 12 h | Within 12 h | Within 12 h |
| | stability duration of the upper, middle and lower layers | Long-term stability: after mixing and sanding for several hours again, the appearance ratio of the upper, middle and lower layers is stable | | | | | |
| | | | | | | | |
| | Compon ent | Embodi ment 7 | Embodi ment 8 | Embodime nt 9 | Embodim ent 10 | Embodi ment 11 | Embodi ment 12 |
| First Soluti on | Several grams of the salt | Sodium chloride 5 g | Sodium chloride 6 g | Sodium chloride 8 g | Sodium chloride 8 g | Sodium chloride 8 g | Sodium chloride 8 g |
| | Total deionize | Deionize d water | Deionize d water | Deionized water | Deionized water | Deioniz ed water | Deionize d water |
| | d water | 25 ml | 30 ml | 40 ml | 40ml | 40 ml | 40 ml |
| | Total water-sol uble pigment | Using toner, blue toner | Using toner, green toner | Using toner, blue toner | Using toner, blue toner | Using toner, green toner | Using toner, red toner |
| | Water-so luble pigment stabilizer | Purchase d from Sensient Technolo gy Corporat ion (China) (Model No. COVAB SORB) | Purchase d from Sensient Technolo gy Corporat ion (China) (Model No. COVAB SORB) | Purchased from Sensient Technolog y Corporatio n (China) (Model No. COVABS ORB) | Purchased from Sensient Technolo gy Corporati on (China) (Model No. COVABS ORB) | Purchas ed from Sensient Technol ogy Corpora tion (China) (Model No. COVAB SORB) | Purchase d from Sensient Technolo gy Corporat ion (China) (Model No. COVAB SORB) |
| | Compon ent | Embodi ment 7 | Embodi ment 8 | Embodime nt 9 | Embodim ent 10 | Embodi ment 11 | Embodi ment 12 |
| Secon d Soluti on | Total water phase solvent | 3-metho xy-3-met hyl-1-but anol 20 ml | 3-metho xy-3-met hyl-1-but anol 30 ml | 3-methoxy -3-methyl-1-butanol 35 ml | 3-methox y-3-meth yl-1-buta nol 12 ml | 3-metho xy-3-me thyl-1-b utanol 20 ml | 3-metho xy-3-met hyl-1-but anol 10 ml |
| | | Dipropyl ene glycol methyl ether 3 ml | Dipropyl ene glycol methyl ether 10 ml | Dipropyle ne glycol methyl ether 15 ml | Dipropyle ne glycol methyl ether 3 ml | Dipropy lene glycol methyl ether 10 ml | Dipropyl ene glycol methyl ether 5 ml |
| | Essence | / | / | / | / | Water-soluble essence, specific ally water-so luble food flavorin g pineappl e essence 10ml | Water-soluble essence, specifica lly water-sol uble peppermi nt essence 10ml |
| | Compon ent | Embodi ment 7 | Embodi ment 8 | Embodime nt 9 | Embodim ent 10 | Embodi ment 11 | Embodi ment 12 |
| Third Soluti on | Oil phase solvent | Caprylic/ capric triglyceri de 10 ml | Caprylic/ capric triglyceri de 20 ml | Isopropyl myristate 20 ml | Isopropyl myristate 25ml | White oil 20 ml | White oil 25 ml |
| | Oil-solub le pigment | Using toner, yellow toner | Using toner, yellow toner | Using toner, yellow toner | Using toner, purple toner | Using toner, purple toner | Using toner, golden toner |
| | Essence | Oil-solub le essence, specifica lly rose oil 10ml | Oil-solub le essence, specifica lly rose oil 20 ml | Oil-solubl e essence, specificall y jasmine oil 15 ml | Oil-solubl e essence, specificall y jasmine oil 10 ml | / | / |
| | Ethanol | Ethanol 2 ml | Ethanol 10 ml | Ethanol 15 ml | Ethanol 2 ml | Ethanol 8 ml | Ethanol 2ml |
| | Oil-solub le pigment stabilizer | Purchase d from Sensient Technolo gy Corporat ion (China) (Model No. COVAB SORB EW) | Purchase d from Sensient Technolo gy Corporat ion (China) (Model No. COVAB SORB EW) | Purchased from Sensient Technolog y Corporatio n (China) (Model No. COVABS ORB EW) | Purchased from Sensient Technolo gy Corporati on (China) (Model No. COVABS ORB EW) | Purchas ed from Sensient Technol ogy Corpora tion (China) (Model No. COVAB SORB EW) | Purchase d from Sensient Technolo gy Corporat ion (China) (Model No. COVAB SORB EW) |
| / | Are there three colors and three layers? | Yes | Yes | Yes | Yes | Yes | Yes |
| / | Layering stability duration | Within 12 h | Within 12 h | Within 12 h | Within 12 h | Within 12 h | Within 12 h |
| / | stability duration of the upper, middle and lower layers | Long-term stability: after mixing and sanding for several hours again, the appearance ratio of the upper, middle and lower layers is stable | | | | | |

The schematic diagram of the sample in Embodiment 1 is shown in FIG. 1. The schematic diagram of the sample in Embodiment 2 is shown in FIG. 2. The final product liquid perfume presents three colors and three layers, and the visual effects of the liquid perfume are richer and attract consumers' attention, so that the consumers' diversified needs can be satisfied, with stable color and uniform volatilization.

Moreover, after use for a period of time, the salt will be adsorbed on the volatile stick (rattan), and a dense white crystalline substance is adhered to the volatile stick above the mouth of the containing bottle, so that another color is added, thus enriching color, increasing different visual effects and improving user experience.

## Claims

1. A liquid perfume having three layers and three colors, comprising a perfume liquor having an upper layer, a middle layer and a lower layer;
**characterized in that**, the upper layer is an oil phase layer comprising an oil phase solvent and an upper-layer pigment which are dissolved into each other;
the middle layer is a water phase layer comprising middle-layer deionized water, a middle-layer water phase solvent and a middle-layer pigment which are dissolved into each other;
the lower layer is a water phase layer comprising lower-layer deionized water, a lower-layer water phase solvent, a lower-layer pigment and salts which are dissolved into each other;
the upper-layer, the middle-layer and the lower-layer each have one kind of different color due to their respective layer pigments;
a density of the lower-layer water phase solvent is greater than a density of the middle-layer water phase solvent, which is greater than a density of the upper-layer oil phase solvent;
a polarity of the lower layer is greater than a polarity of the middle layer, which is greater than a polarity of the upper layer; and
at least one of the upper layer, the middle layer and the lower layer has an essence.

2. The liquid perfume according to claim 1, **characterized in that** in the upper layer, the oil phase solvent comprises at least one of isoparaffin, dipropyleneglycol methyl ether acetate, caprylic/capric triglyceride, isopropyl myristate and white oil.

3. The liquid perfume according to claim 1, **characterized in that** the middle-layer water phase solvent comprises 3-methoxy-3-methyl-1-butanol, and further comprises at least one of dipropyleneglycol methyl ether ethyl ester, dipropylene glycol methyl ether, dipropyleneglycol dimethyl ether and ethanol.

4. The liquid perfume according to claim 1, **characterized in that** the lower-layer water phase solvent comprises at least one of 3-methoxy-3-methyl-1-butanol, dipropyleneglycol methyl ether ethyl ester, dipropylene glycol methyl ether, dipropyleneglycol dimethyl ether and ethanol.

5. The liquid perfume according to claim 1, **characterized in that** the salts comprises at least one of potassium chloride, anhydrous sodium sulfate and sodium chloride.

6. The liquid perfume according to claim 1, **characterized in that** the essence comprises a natural essence and/or a synthetic essence.

7. The liquid perfume according to claim 1, **characterized in that** the upper layer further comprises an oil-soluble pigment stabilizer, and the oil-soluble pigment stabilizer is dissolved in the oil phase solvent to prevent the upper-layer pigment from fading and discoloring.

8. The liquid perfume according to claim 1, **characterized in that** the middle layer further comprises a water-soluble pigment stabilizer, and the water-soluble pigment stabilizer is dissolved in the middle-layer water phase solvent to prevent the middle-layer pigment from fading and discoloring;
and/or, the lower layer further comprises a water-soluble pigment stabilizer, and the water-soluble pigment stabilizer is dissolved in the lower-layer water phase solvent to prevent the lower-layer pigment from fading and discoloring.

9. The liquid perfume according to claim 7, **characterized in that** the oil-soluble pigment stabilizer comprises at least one of octyl methoxycinnamate, butyl methoxydibenzoylmethane and octyl salicylate.

10. The liquid perfume according to claim 8, **characterized in that** the water-soluble pigment stabilizer comprises at least one of octyl methoxycinnamate, butyl methoxydibenzoylmethane, PPG-26-buteth-26, octyl salicylate and PEG-40-hydrogenated castor oil.

11. The liquid perfume according to claim 1, **characterized in that** the liquid perfume comprises a containing bottle for containing the perfume liquor.

12. The liquid perfume according to claim 11, **characterized in that** the containing bottle has an opening at a top of the containing bottle, and the liquid perfume further comprises a volatile stick which is inserted into the opening and immersed in the lower layer of the perfume liquor;
the containing bottle is made of a transparent material.

13. A manufacturing method for the liquid perfume having three layers and three colors of claim 1, comprising the following steps:
step 1:
dissolving a salt in total deionized water, adding a water-soluble pigment, and mixing uniformly to obtain a first solution; and
using a total water phase solvent as a second solution;
step 2:
mixing an oil phase solvent with an oil-soluble pigment uniformly to obtain a third solution, the water-soluble pigment and the oil-soluble pigment having different colors;
if the essence is water-soluble, the essence is added in the step 1; and, if the essence is oil-soluble, the essence is added in the step 2;
step 3:
according to the volume required by each layer of the final product liquid perfume, determining the volume required by each of the first solution, the second solution and the third solution, mixing and shaking the first solution and the second solution uniformly, standing to clarification, adding the third solution and further mixing and shaking uniformly, and standing to clarification to obtain the upper layer, the middle layer and the lower layer;
a total deionized water comprises the middle-layer deionized water and the lower-layer deionized water;
a total water phase solvent comprises the middle-layer water phase solvent and the lower-layer water phase solvent;
the water-soluble pigment finally enters the middle layer and the lower layer, that is, the water-soluble pigment comprises part of the middle-layer pigment and part of the lower-layer pigment; and
the oil-soluble pigment finally enters the upper layer and the middle layer, that is, the oil-soluble pigment comprises part of the upper-layer pigment and part of the middle-layer pigment.

14. The manufacturing method according to claim 13, **characterized in that** during preparation of the first solution in the step 1, after the water-soluble pigment is added, a water-soluble pigment stabilizer for preventing the water-soluble pigment from fading and discoloring is also added; and
in the step 3, after the oil phase solvent and the oil-soluble pigment are added, an oil-soluble pigment stabilizer for preventing the oil-soluble pigment from fading and discoloring is also added.

15. The manufacturing method according to claim 13, **characterized in that** components by volume percentage are:
25ml - 40ml of total deionized water;
5g - 8g of sodium chloride;
the total water phase solvent comprises:
10 ml - 35 ml of 3-methoxy-3-methyl-1-butanol;
3 ml - 15 ml of dipropylene glycol methyl.

16. The manufacturing method according to claim 15, **characterized in that** the essence is oil-soluble and added during the preparation of the third solution in the step 2, and the third solution further comprises the following components by volume percentage:
2 ml - 15 ml of ethanol;
10 ml - 25 ml of the essence.

17. The manufacturing method according to claim 15, **characterized in that** the oil phase solvent is 10 ml - 25 ml of isoparaffin, or dipropyleneglycol methyl ether acetate, or caprylic/capric triglyceride, or isopropyl myristate, or white oil.
